# EUROPEAN PATENT APPLICATION

(11) **EP 4 609 771 A1**
(43) Date of publication of application: **03.09.2025**
(21) Application number: 24160182.2
(22) Date of filing: 28.02.2024
(51) Int. Cl.: A47K 13/12, A47K 13/24, A61B 5/00, A61B 5/01, A61B 5/0205, A61B 5/024, A61B 5/0245, A61B 5/318, G01G 19/44

(54) **SMART TOILET SEAT ASSEMBLY, LOAD SENSOR, AND FIXING ARRANGEMENT**

(71) Applicant: Stichting IMEC Nederland, 6708 WH Wageningen (NL)
(72) Inventor: Wentink, Eva, 5591 MT Heeze (NL); Verbiest, Vera, 6721 SV Bennekom (NL); Young, David, 5645 JC Eindhoven (NL); van Hardeveld, Remco, 3901 KT Veenendaal (NL)
(74) Representative: Körfer, Thomas

(57) **Abstract**

A smart toilet seat assembly (100) for a toilet bowl for measuring physiological parameters of a user is provided. The smart toilet seat assembly comprises a seat (101) comprising a plurality of physiological sensors (102, 103, 104), each of the plurality of physiological sensors (102, 103, 104) being configured to generate at least one physiological parameter measurement data of the user. The smart toilet seat assembly further comprises a plurality of load sensors (108) configured to support the seat (101) on the toilet bowl when used by the user and further to collectively generate a weight measurement data of the user. Moreover, the smart toilet seat assembly comprises a fixing arrangement (106) configured to attach a lid (107) to the seat (101) and further to attach the smart toilet seat assembly to the toilet bowl. In this regard, the fixing arrangement (106) is configured to transfer a weight of the lid (107) exclusively to the toilet bowl and further to transfer a weight applied on the seat (101) exclusively to the plurality of load sensors (108).

## Description

The invention relates to sensor integration in smart health toilet seats, especially for measuring health related parameters of a user.

Generally, regularly measuring health parameters may be required for many diagnosed patients as well as for people at risk. A toilet seat can be used as an easy, non-obtrusive way of measuring health parameters. People with a condition, for instance inflammatory bowel disease (IBD), can also be benefitted from this way of tracking parameters, since the measurements can be used to notify the condition of their health, which may allow them to take appropriate actions. This may prevent worsening of conditions, and may allow monitoring of diseases and of health, or tracking of medication.

For example, the document US 2019/0231271 A1 discloses a toilet seat for medical analysis of a seated individual. In this regard, the toilet seat comprises force sensors for measuring changes in the apparent weight of the individual, where the seat is secured to the surface via floating hinges. However, the seat cover is connected to the hinges in the conventional manner, which may affect the measurement accuracy of the force sensors.

Accordingly, an object of the invention is to provide a smart toilet seat assembly, a load sensor, and a fixing arrangement for measuring health related parameters of the user, whereby improving the measurement accuracy of the integrated sensors.

The object is solved by the features of the first independent claim for the smart toilet seat assembly, by the features of the second independent claim for the load sensor, and by the features of the third independent claim for the fixing arrangement. The dependent claims contain further developments.

According to a first aspect of the invention, a smart toilet seat assembly for a toilet bowl for measuring physiological parameters of a user is provided. The smart toilet seat assembly comprises a seat comprising a plurality of physiological sensors, each of the plurality of physiological sensors being configured to generate at least one physiological parameter measurement data of the user.

The smart toilet seat assembly further comprises a plurality of load sensors configured to support the seat on the toilet bowl when used by the user and further to collectively generate a weight measurement data of the user. Moreover, the smart toilet seat assembly comprises a fixing arrangement configured to attach a lid to the seat and further to attach the smart toilet seat assembly to the toilet bowl.

In this regard, the fixing arrangement is configured to transfer a weight of the lid exclusively to the toilet bowl and further to transfer a weight applied on the seat exclusively to the plurality of load sensors.

Advantageously, by means of the sensor integration in the toilet seat, an easy and a non-obtrusive way of measuring health parameters can be achieved. Furthermore, by transferring the weight of the lid directly to the toilet bowl and by transferring the weight applied on the toilet seat exclusively to the load sensors, a high measurement accuracy of the sensors, especially of the load sensors, can be achieved.

Preferably, the plurality of physiological sensors are arranged on a top surface of the seat being in physical contact with the user. Additionally or alternatively, at least one sensor of the plurality of physiological sensors is configured to be in contact with the user, especially in physical contact with the user.

Preferably, the plurality of load sensors are arranged on a bottom surface of the seat being in contact with the toilet bowl.

Advantageously, for example, the sensors that require a physical contact to the user can be integrated in the top surface of the seat and the load sensors can be integrated in the bottom surface of the seat to ensure an accurate readout of the weight applied on the seat.

Preferably, the plurality of physiological sensors comprise at least one electrocardiogram (ECG) sensor, at least one bio-impedance (BioZ) sensor, at least one photoplethysmography (PPG) sensor, and at least one temperature sensor.

In this regard, the at least one temperature sensor may not be in direct and/or physical contact with the user. For instance, the at least one temperature sensor may be arranged on the seat especially in a close proximity, e.g., about 30cm or less, in relation to the user.

Additionally or alternatively, the at least one physiological parameter measurement data comprises an electrocardiogram measurement data or a bio-impedance measurement data or a photoplethysmography measurement data or a temperature measurement data of the user.

For instance, the sensors may provide physiological parameter measurement data, which can be processed to extract important health related features such as heart rate, heart rate variability, blood pressure changes including the pulse transit time, pulse arrival time, and pre-ejection period, absolute weight, weight change over time, urine or stool condition, weight loss per toilet session including urine flow rate, the amount of urine, and the amount of stool, bowel movement or pressure, number of toilet sessions, respiration rate, type of respiration, core temperature, position on the seat, oxygen saturation, body composition, inflammation, and so on.

Preferably, the seat comprises a switching arrangement configured to switch on or switch off the plurality of physiological sensors and/or the plurality of load sensors based on a presence or an absence of the user, respectively.

For instance, the switching arrangement may comprise a capacitive touch sensor that may detect the presence of the user, especially when the user is sitting on the toilet seat, and may initiate the sensors for sensing and measuring upon detecting the user. Advantageously, the switching arrangement may facilitate a power saving scheme in order not to consume too much power when the toilet seat is not in use.

Preferably, the lid comprises a receiving unit configured to receive the at least one physiological parameter measurement data from the plurality of physiological sensors especially arranged on the top surface of the seat and/or the weight measurement data from the plurality of load sensors especially arranged on the bottom surface of the seat.

For example, the lid, especially the receiving unit of the lid, may be connected to the seat, especially to the sensors integrated in the seat, through a wired data link or a wireless data link, and the lid may receive the measurement data from the sensors through the wired data link or wirelessly.

Preferably, the lid comprises a processing unit configured to process the at least one physiological parameter measurement data and/or the weight measurement data and further to extract one or more physiological parameters of the user from the at least one physiological parameter measurement data and/or the weight measurement data. Advantageously, for example, in-situ measurement data processing and feedback processing can be achieved.

Preferably, the lid comprises a display unit configured to display a notification corresponding to the extracted one or more physiological parameters of the user. Advantageously, for example, user experience can be significantly enhanced.

Preferably, the lid comprises a transmitting unit configured to transmit the one or more physiological parameters of the user to a remote device and/or to a cloud storage, preferably wirelessly. Advantageously, for example, ex-situ feedback processing, especially for the assessment of health professionals, can be achieved.

Preferably, the seat comprises a supply unit configured to provide an electrical supply to the plurality of physiological sensors and/or the plurality of load sensors. Advantageously, for example, the proper operation of the sensors integrated in the toilet seat can be ensured.

Preferably, the lid comprises a charging unit operably coupled to the supply unit of the seat, the charging unit being configured to transmit an electrical power to the supply unit in order to charge or recharge the supply unit. Advantageously, in-situ charging or recharging of the toilet seat in order to ensure the operation of the sensors integrated in the toilet seat can be achieved.

According to a second aspect of the invention, a load sensor is provided for generating a weight measurement data in a smart toilet seat assembly according to the first aspect of the invention. The load sensor comprises a holding segment configured to attach the load sensor to a seat of the smart toilet seat assembly, a foot segment configured to be in contact with a toilet bowl when used by a user, and a sensor body comprising a load cell, the sensor body being arranged between the holding segment and the foot segment.

In this regard, the foot segment comprises a crest configured to protrude towards the holding segment through the sensor body. Furthermore, the holding segment comprises a magnet configured to align the crest of the foot segment through the sensor body and further to attach the foot segment with the holding segment.

Preferably, the load cell is a strain gauge load cell.

Advantageously, for example, the weight applied on the toilet seat can be measured with a high accuracy and in a cost-effective manner. Furthermore, the magnetic attachment between the holding segment and the foot segment may ensure a precise alignment of the segments when the sensor is assembled in the toilet seat. The magnetic attachment may additionally ensure cleanability and/or ease of replacing parts when needed.

Preferably, a readout circuit may be integrated in the load sensor for reading out the sensor signal. Advantageously, by integrating the readout circuit in the sensor enclosure, sensor data degradation due to path loss can be significantly reduced.

According to a third aspect of the invention, a fixing arrangement is provided for attaching a smart toilet seat assembly according to the first aspect of the invention to a toilet bowl. In this regard, the fixing arrangement comprises at least one hinge.

The at least one hinge comprises a hinge body configured to attach the at least one hinge to the toilet bowl. The at least one hinge further comprises a first bracket directly coupled to the hinge body, wherein the first bracket is configured to attach a lid of the smart toilet seat assembly to the hinge body in order to transfer a weight of the lid exclusively to the toilet bowl and further to rotate the lid with respect to the hinge body.

Moreover, the at least one hinge comprises a second bracket coupled to the hinge body via a coupler, wherein the second bracket is configured to attach a seat of the smart toilet seat assembly to the hinge body via the coupler and further to rotate the seat with respect to the hinge body. In this regard, the coupler is configured to move the seat along a vertical axis based on a weight applied on the seat.

Advantageously, by transferring the weight of the lid directly to the toilet bowl and by transferring the weight applied on the toilet seat exclusively to the load sensors, a high measurement accuracy of the sensors, especially of the load sensors, can be achieved.

Preferably, the coupler comprises a limiting member configured to control the movement of the seat along the vertical axis.

Preferably, the first bracket comprises a first damping mechanism configured to control the rotational movement of the lid with respect to the hinge body. Additionally or alternatively, the second bracket comprises a second damping mechanism configured to control the rotational movement of the seat with respect to the hinge body.

Advantageously, for example, a softer, slower, and quieter movement of the lid and/or the seat can be facilitated, which may increase the comfort and ease of use.

Exemplary embodiments of the invention are now further explained with respect to the drawings by way of example only, and not for limitation. In the drawings:
- Fig. 1A: shows a top view of an exemplary embodiment of the smart toilet seat assembly according to an aspect of the invention;
- Fig. 1B: shows a bottom view of the smart toilet seat assembly of Fig. 1A;
- Fig. 2A: shows a top view of the lid of the smart toilet seat assembly according to an aspect of the invention;
- Fig. 2B: shows a bottom view of the lid of the smart toilet seat assembly according to an aspect of the invention;
- Fig. 3: shows an exemplary operation of the smart toilet seat assembly according to an aspect of the invention;
- Fig. 4: shows an exemplary charging scheme of the smart toilet seat assembly according to an aspect of the invention;
- Fig. 5: shows an exemplary embodiment of the hinge according to an aspect of the invention;
- Fig. 6A: shows a front view of the hinge of Fig. 5;
- Fig. 6B: shows a back view of the hinge of Fig. 5;
- Fig. 7A: shows an exemplary embodiment of the load sensor according to an aspect of the invention;
- Fig. 7B: shows an exploded view of the load sensor of Fig. 7A;
- Fig. 8A: shows a front view of the load sensor of Fig. 7A; and
- Fig. 8B: shows an inside view of a segment of the load sensor of Fig. 7A from bottom.

Reference will now be made in detail to the embodiments of the present invention, examples of which are illustrated in the accompanying drawings. However, the following embodiments of the present invention may be variously modified and the range of the present invention is not limited by the following embodiments.

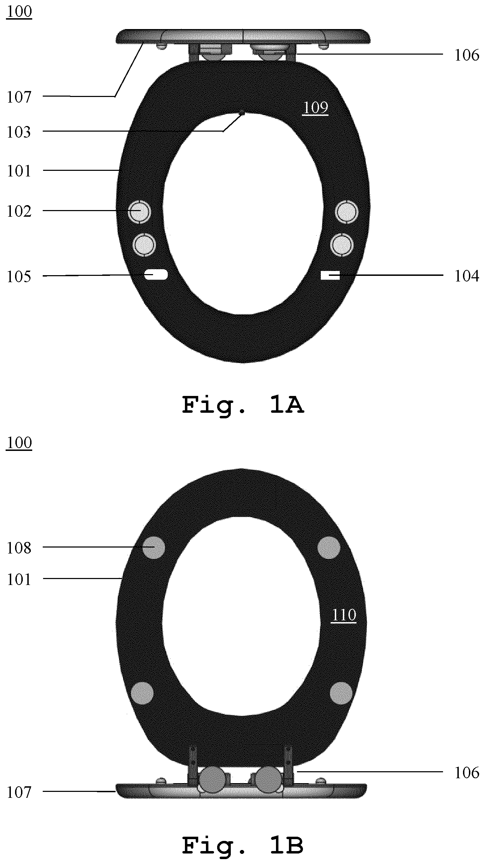

In Fig. 1A and Fig. 1B, an exemplary embodiment of the smart toilet seat assembly 100 is illustrated. In particular, Fig. 1A shows a top view of the smart toilet seat assembly 100. The smart toilet seat assembly 100 may comprise a seat 101 and a lid 107. The seat 101 may comprise a number of ECG and/or BioZ electrodes 102 on a top surface 109 of the seat 101, exemplarily four ECG and/or BioZ electrodes, for measuring ECG and BioZ of the user of the seat 101 while being in physical contact with the user.

For example, in order to measure ECG and/or BioZ of the user, two source electrodes may be provided to excite from two different measurement locations on the user, and two receiver electrodes may be provided to respectively measure response from the two different measurement locations to realize a tetrapolar or four-electrode measurement technique, especially to accurately generate the ECG measurement data and/or the BioZ measurement data.

For instance, the ECG and/or BioZ electrodes 102 may be operated with multiple frequencies as well as with a single frequency. For instance, a frequency division can be implemented for parallel acquisition of the ECG data (low frequency) and the BioZ data (high frequency).

The seat 101 may further comprise a PPG sensor 104 on the top surface 109 of the seat 101. The PPG sensor 104 may provide measurement data related to the heart rate, oxygen saturation, blood pressure variables, and so on while being in physical contact with the user. The PPG sensor 104 may be arranged on the top surface 109 of the seat 101 to be in physical contact with the leg of the user.

For example, the PPG sensor 104 may comprise different colors of light-emitting diodes (LEDs). For instance, the PPG sensor 104 can be a PPG module or may comprise separate LED and/or photodiodes (PDs). Moreover, the PPG sensor 104 may comprise or be a singular PPG sensor or an array of PPG sensors.

For example, the seat 101 may comprise an IR temperature sensor 103 that may measure core temperature of the user. In this regard, the IR temperature sensor 103 may be arranged on the seat 101 such that the IR temperature sensor 103 can perform rectal temperature measurement of the user while using the seat 101, thereby improving the quality and the reliability of the core temperature measurement. In this regard, the IR temperature sensor 103 may comprise or be a singular IR sensor or an array of IR sensors.

For example, the seat 101 may comprise a capacitive touch sensor 105 arranged on the top surface 109 of the seat 101. The capacitive touch sensor 105 may detect when the user is sitting on the seat 101, and may immediately start all sensors for commencing the measurements. In case the presence of the user cannot be detected by the capacitive touch sensor 105, e.g., the user is in standing position and/or not using the seat, the capacitive touch sensor 105 may switch off the sensors (idle state).

For example, the lid 107 may be attached to the seat 101 via a fixing arrangement 106 comprising two hinges. The fixing arrangement 106 may also attach the smart toilet seat assembly 100 to a toilet bowl (not shown).

Fig. 1B shows a bottom view of the smart toilet seat assembly 100. It can be seen that the seat 101 may comprise a number of load sensors 108, exemplarily four load sensors, arranged on a bottom surface 110 of the seat 101, especially between the seat 101 and the toilet bowl. The load sensors 108 may collectively measure the weight applied on the seat 101, e.g., the weight of the user sitting on the seat 101.

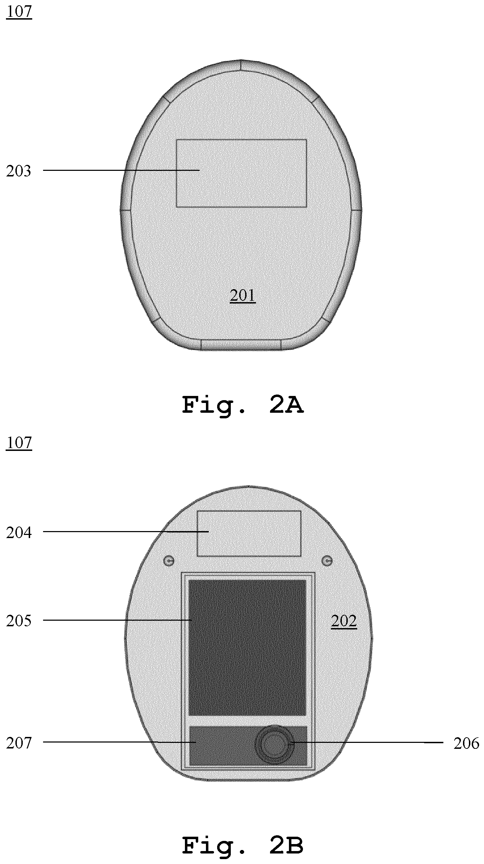

In Fig. 2A and Fig. 2B, an exemplary embodiment of the lid 107 is illustrated is illustrated. In particular, Fig. 2A shows a top view of the lid 107. For example, the lid 107 may comprise a first display 203 arranged on a top surface 201 of the lid 107. The first display 203 may display one or more notifications regarding one or more health related status of the user.

Fig. 2B shows a bottom view of the lid 107. For example, the lid 107 may comprise a second display 204 arranged on a bottom surface 202 of the lid 107. The second display 204 may display one or more notifications regarding one or more health related status of the user.

The lid 107 may further comprise a charging unit 205, a processing unit 207, and a wireless antenna 206 arranged on the bottom surface 202 of the lid 107. The charging unit 205 may charge or recharge the seat 101 via the wireless antenna 206, while the processing unit 207 may receive the measurement data from the seat 101 and may process the measurement data to derive one or more health related parameters.

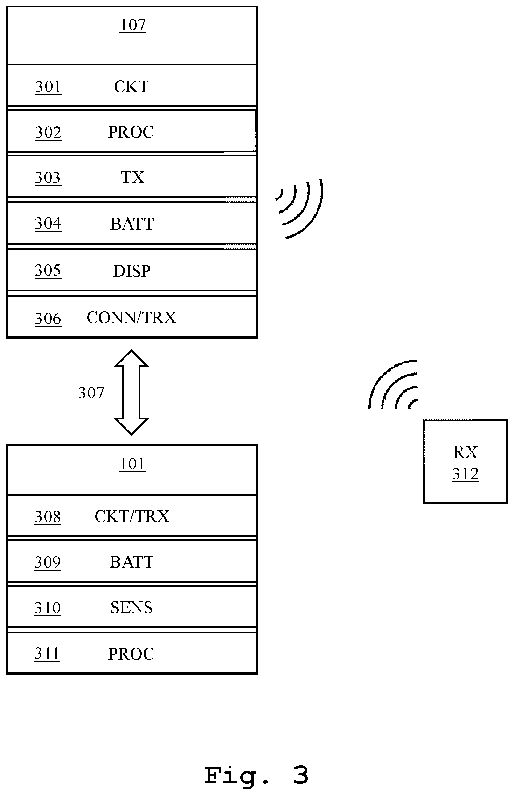

In Fig. 3, an exemplary operation of the smart toilet seat assembly 100 is illustrated. In particular, Fig. 3 shows a block representation of the components of the smart toilet seat assembly 100.

For example, the seat 101 may comprise a transceiver block 308, a battery 309, a sensor block 310, and a processing block 311.

For example, the sensor block 310 may comprise the ECG and/or BioZ sensors 102, the IR temperature sensor 103, the PPG sensor 104, the capacitive touch sensor 105, and the load sensors 108. The battery 309 may be a rechargeable battery, which may provide electrical power to the sensor block 310.

For example, the processing block 311 may comprise a processor, which may regulate the timing operation of the sensor block 310, e.g., to regulate the timing operation of the ECG and/or BioZ sensors 102, the IR temperature sensor 103, the PPG sensor 104, the capacitive touch sensor 105, and the load sensors 108.

For example, the transceiver block 308 may transmit the measurement data, either wired or wirelessly, from the sensor block 310, especially from the ECG and/or BioZ sensors 102, the IR temperature sensor 103, the PPG sensor 104, and the load sensors 108, to the lid 107. In addition, the transceiver block 308 may receive electrical signals, either wired or wirelessly, from the lid 107 in order to charge or recharge the battery 309. Additionally, the electrical signals communicated between the lid 107 and the transceiver block 308 may comprise control signals for turning on/off the processing block 311, switching on/off certain signals and/or sensors, and so on.

Additionally or alternatively, the seat 101 may comprise a charging interface, which can be connected to a power socket to allow direct charging or recharging of the battery 309.

Additionally or alternatively, the processing block 311 may process the sensor data and may derive one or more health related parameters from the sensor data. In this regard, the transceiver block 308 may transmit the raw sensor data and/or the processed one or more health related parameters to a remote device 312, especially wirelessly.

The remote device 312 can be a wireless receiving device, such as cell phones, PDAs, or can be a signal analysis device, such as VNAs, personal computers. Additionally or alternatively, the remote device 312 may be a remote storage device, such as a network storage or a cloud storage.

For example, the lid 107 may comprise a charging circuit block 301, especially a wireless charging circuit, a processing block 302, a transmitter block 303, especially a wireless transmitter, a battery pack 304, a display block 305, and a connection block 306.

For instance, the charging circuit block 301 may comprise a wireless charging antenna, which may be covered with a flexible material, such as silicon, and be pushed out to make a proper contact with the seat 101 when the lid 107 is closed. In this regard, the charging circuit block 301, by means of the battery pack 304 and the wireless charging antenna, may provide wireless charging to the seat 101, especially to the battery 309 of the seat 101.

For example, the lid 107 can be independently removed or detached from the smart toilet seat assembly 100. As such, the lid 107 can be used as a wireless power bank for the seat 101, and can be detached and the battery pack 304 can be recharged when needed. In this regard, the lid 107 may comprise a charging interface, e.g., an USB interface, which can be connected to a power socket for charging or recharging of the battery pack 304.

For example, the connection block 306 may comprise wired or wireless link 307, and may receive the measurement data from the ECG and/or BioZ sensors 102, the IR temperature sensor 103, the PPG sensor 104, and the load sensors 108 via the link 307. In this regard, the processing block 302 may comprise a processor, which may process the sensor data and may derive one or more health related parameters from the measurement data.

For example, the processing block 302 may display one or more health related status to the user via the display block 305. The display block 305 may comprise one or more displays, e.g., as described along Fig. 2A and Fig. 2B.

For example, the transmitter block 303 may transmit the raw measurement data and/or the processed one or more health related parameters to the remote device 312, especially wirelessly.

Although not shown, the seat 101 and/or the lid 107 may comprise one or more indicators, e.g., LED indicators, indicating the status of the battery or battery pack, the status of one or more health related parameters, a cloud connection, the uploading of measurement data or new measurement data, errors, calibration of the sensors, and so on.

Although not shown, the seat 101 and/or the lid 107 may comprise a user identification detecting module, e.g., a RFID detection module, which may detect a specific user based on the specific user identification, e.g., user specific RFID tags. Accordingly, the measurement data can be assigned or be regarded to a specific user of the smart toilet seat assembly 100, especially in multi-user scenarios.

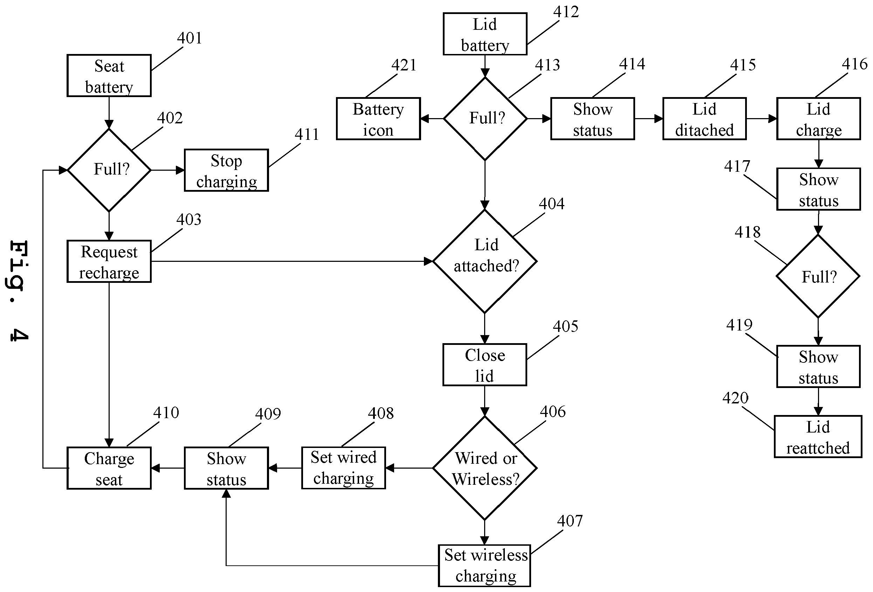

In Fig. 4, an exemplary charging scheme of the smart toilet seat assembly 100 is illustrated. For example, during the charging step 401 of the battery 309 of the seat 101, a check 402 may be performed to obtain the charge status of the battery 309. If the battery 309 of the seat requires charging, a recharge request 403 may be indicated, e.g., via one or more LED indicators.

Upon receiving the recharge request 403, a further check 404 may be performed to ensure that the lid 107 is attached to the seat 101. If the lid 107 is attached to the seat 101, the lid 107 may be closed in the next step 405, e.g., automatic closure of the lid 107, to allow charge transfer from the battery pack 304 of the lid 107 to the battery 309 of the seat 101.

In this regard, a further check 406 may be performed to decide the mode of charge transfer. In case of wireless charging 407, the wireless charging link between the lid 107 and the seat 101 may be set. Alternatively, the seat 101 may be directly charged 408 via a power socket or via a wired link between the lid 107 and the seat 101.

In this regard, a status 409 regarding the mode of charge transfer of the previous step 407 or 408 and/or the status of the battery 309 may be indicated, e.g., via one or more LED indicators. In a consecutive step 410, the charging or recharging of the seat 101 may be initiated. After reaching the charge capacity of the battery 309, i.e., after repeating the check 402 and if the charge capacity of the battery 309 of the seat 101 is full, the charging of the battery 309 may be stopped in the next step 411.

Alternatively, if the charge capacity of the battery 309 of the seat 101 is already full, e.g., resulted during the check 402 to obtain the charge status of the battery 309, the process flow may omit the steps 403 to 410, and may initiate the step 411 to stop charging.

For example, during the charging step 412 of the battery pack 304 of the lid 107, a check 413 may be performed to obtain the charge status of the battery pack 304 and the status 414 may be indicated, e.g., on the displays or via one or more LED indicators. Afterwards, the user may detach 415 the lid 107 from the seat 101 and may connect to a power socket for charging 416, and the charging status 417 may be indicated, e.g., on the displays or via one or more LED indicators.

A further check 418 regarding the charge status of the battery pack 304 may be performed, and upon reaching the charge capacity of the battery pack 304 the charge status 419 of the battery pack 304 may be indicated, e.g., on the displays or via one or more LED indicators. Afterwards, the user may reattach 420 the lid 107 to the seat 101.

Alternatively, if the charge capacity of the battery pack 304 of the lid 107 is already full, e.g., resulted during the check 413 to obtain the charge status of the battery pack 304, the process flow may omit the steps 414 to 420, and may initiate the step 421 to indicate that the battery pack 304 is at full capacity, e.g., on the displays or via one or more LED indicators.

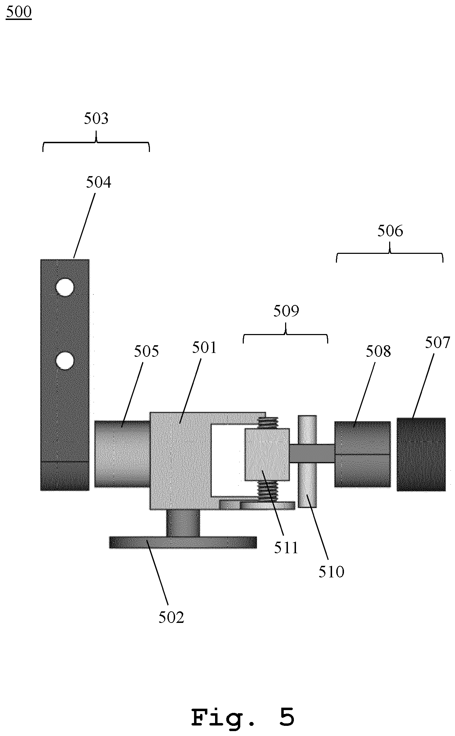

In Fig. 5, an exemplary embodiment of the hinge 500 of the fixing arrangement is illustrated. In particular, Fig. 5 shows a left side hinge 500 of the fixing arrangement. The fixing arrangement may further comprise a right side hinge, and the operation of the right side hinge may be analogous to the left side hinge 500.

For example, the hinge 500 may comprise a hinge body that may attach the hinge 500 to the toilet bowl. The hinge body may comprise a main hinge body 501 and a foot 502, where the foot 502 can be detached from the toilet bowl in order to detach the hinge 500 from the toilet bowl.

For example, the hinge 500 may comprise a first bracket 503 coupled to the main hinge body 501. The first bracket 503 may comprise a lid attachment 504 that may attach the lid 107 of the smart toilet seat assembly 100 to the main hinge body 501. The lid attachment 504 may be attached to the main hinge body 501 in a rotatable manner to facilitate rotational movement of the lid 107 for opening or closing.

The first bracket 503 may further comprise a first damping mechanism 505 arranged between the lid attachment 504 and the main hinge body 501. In other words, the lid attachment 504 may be attached to the main hinge body 501 via the first damping mechanism 505. In this regard, the first damping mechanism 505 may provide a frictional force to control the rotational movement of the lid attachment 504 along the main hinge body 501, thereby facilitating a soft close of the lid 107.

For example, the hinge 500 may comprise a second bracket 506 coupled to the main hinge body 501 via a coupler 509. The second bracket 506 may comprise a seat attachment 507 that may attach the seat 101 of the smart toilet seat assembly 100 to the main hinge body 501 via the coupler 509. The seat attachment 507 may be attached to the main hinge body 501 via the coupler 509 in a rotatable manner to facilitate rotational movement of the seat 101.

The second bracket 506 may further comprise a second damping mechanism 508 arranged between the seat attachment 507 and the coupler 509. In other words, the seat attachment 507 may be attached to the coupler 509 via the second damping mechanism 508. In this regard, the second damping mechanism 508 may provide a frictional force to control the rotational movement of the seat attachment 507 along coupler 509, respectively along the main hinge body 501, thereby facilitating a soft close of the seat 101.

The coupler 509 may comprise a fixation rod 510 and the second bracket 506 may be coupled to the fixation rod 510 such that the second bracket 506 can be moved along a vertical axis, especially along the fixation rod 510, in order to provide a floating arrangement of the seat 101. The coupler 509 may additionally comprise a limiting member 511 that may control the vertical movement of the second bracket 506 along the fixation rod 510.

As such, the first bracket 503 may be directly coupled to the main hinge body 501 and the weight of the lid 107 may be transferred directly to the toilet bowl via the main hinge body 501 and the foot 502. On the other hand, the second bracket 506 may float along the main hinge body 501, especially along the fixation rod 510, in order to prevent the weight applied on the seat 101 to be transferred to the hinge 500. Accordingly, for the smart toilet seat assembly 100, the weight applied on the seat 101, e.g., the weight of the user, can be exclusively transferred to the load sensors 108.

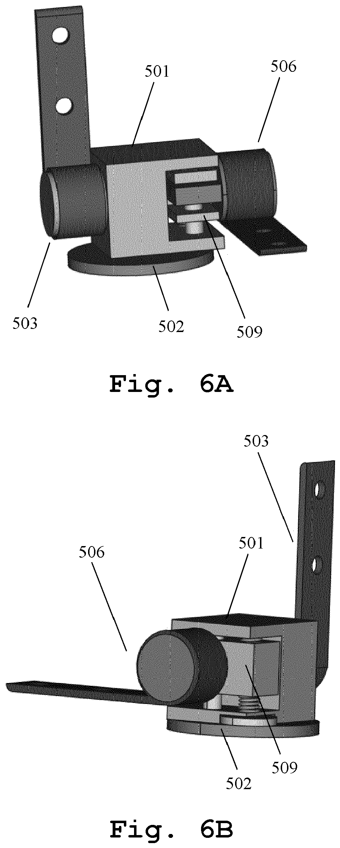

Fig. 6A shows a front view of the hinge 500 and Fig. 6B shows a back view of the hinge 500. It is to be noted that the coupler 509 may only comprise the fixation rod 510 and the vertical motion of second bracket 506, i.e., the vertical motion of the seat 101, can be controlled by providing a fixed gap in the first damping mechanism 505 of the first bracket 503, which may automatically fix the vertical range.

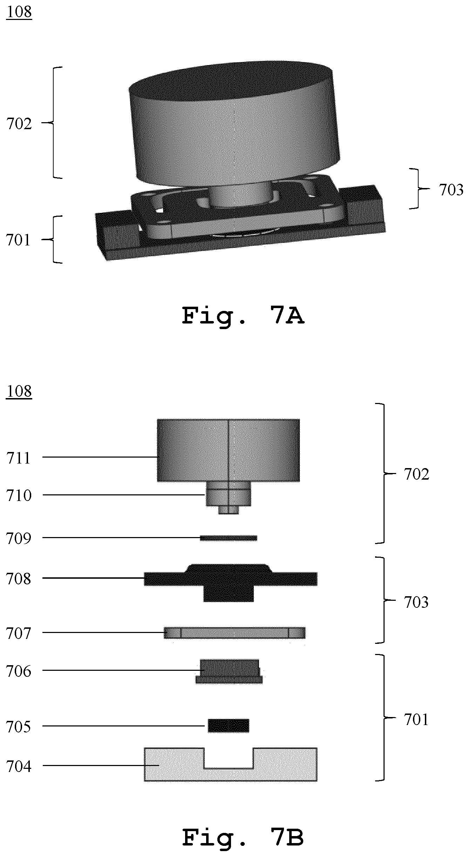

In Fig. 7A and Fig. 7B, an exemplary embodiment of the load sensor 108 is illustrated. For example, the load sensor 108 may comprise a holding segment 701 that may attach the load sensor 108 to the seat 101 of the smart toilet seat assembly 100. The load sensor 108 may further comprise a foot segment 702 that may be in contact with the toilet bowl when the seat 101 is used by the user. Moreover, the load sensor 108 may comprise a sensor body 703 arranged between the holding segment 701 and the foot segment 702. For example, the holding segment 701 and the foot segment 702 may completely cover the sensor body 703.

Fig. 7B shows an exploded view of the load sensor 108. For example, the holding segment 701 may comprise a base 704, a magnet 705, and a readout circuit 706, e.g., in the form of a PCB. For instance, the base 704 may comprise a groove and the magnet 705 may be arranged in the groove of the base 704.

For example, the sensor body 703 may comprise a load cell 707, e.g., a strain gauge load cell, operably coupled to the readout circuit 706 of the holding segment 701. For instance, the readout circuit 706 of the holding segment 701 may be connected to other readout circuits of neighboring load cells, e.g., through a further groove of the base 704 of the holding segment 701. For example, the base 704 may comprise a further groove corresponding to the load cell 707 such that the load cell 707 can be arranged within the base 704 of the holding segment 701.

For example, the sensor body 703 may further comprise a cover 708 that may fully cover the side of the sensor body 703 opposite to the base 704 of the holding segment 701.

The foot segment 702 may comprise a foot base 711 and a crest 710 protruding from the foot base 711. For example, the foot base 711 may have a larger diameter than the diameter of the crest 710 in order to support the seat 101 on the toilet bowl.

In this regard, each of the cover 708, the load cell 707, and the readout circuit 706 may comprise a central hole, approximately of the diameter of the crest 710 such that the crest 710 can pass through the cover 708, the load cell 707, and the readout circuit 706, and can make a magnetic contact with the magnet 705. For example, a rubber ring 709, e.g., as an additional or optional component of the foot segment 702, may be arranged along the diameter of the crest 710 in order to facilitate an elastic contact between the foot base 711 and the cover 708 as well as to prevent dirt ingress.

As such, the magnet 705 may guide the crest 710, thereby guiding the foot segment 702 and may hold the foot segment 702 appropriately, especially during the assembly of the load sensor 108.

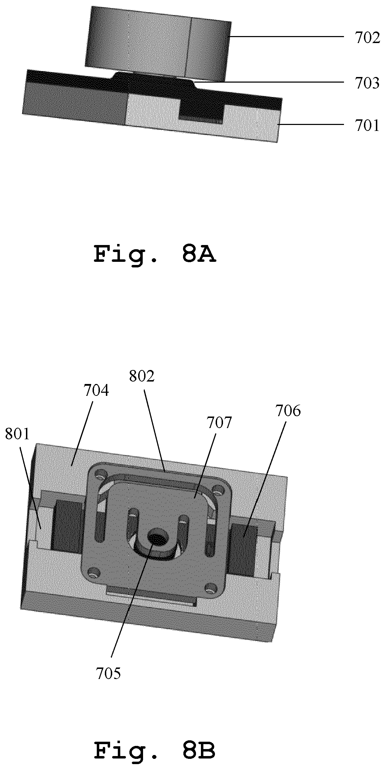

Fig. 8A shows a front view of the load sensor 108 and Fig. 8B shows an inside view of the load sensor 108 from bottom. In particular, Fig. 8B shows the load cell 707 inside of the base 704 of the holding segment 701. It can be seen that the groove 801 for the readout circuit or the PCB 706 may allow for a sideway extension of the electrical connection between neighboring load sensors, especially between neighboring readout circuits. It can further be seen that the groove 802 may allow the proper positioning of the load cell 707 inside of the base 704 of the holding segment 701.

It is important to note that, in the description as well as in the claims, the word "comprising" does not exclude other elements or steps and the indefinite article "a" or "an" does not exclude a plurality. A single element or other unit may fulfill the functions of several entities or items recited in the claims.

It should be understood that the term "and/or" used in the specification and the appended claims of this application refers to any combination and all possible combinations of one or more associated listed items, and includes these combinations.

It should also be understood that the word "connected" or "coupled" implies that the elements may be directly connected together or may be coupled through one or more intervening elements. Moreover, the disclosure with regard to any of the aspects is also relevant with regard to the other aspects of the disclosure.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

## Claims

1. A smart toilet seat assembly (100) for a toilet bowl for measuring physiological parameters of a user, the smart toilet seat assembly comprises:
a seat (101) comprising a plurality of physiological sensors (102, 103, 104), each of the plurality of physiological sensors (102, 103, 104) being configured to generate at least one physiological parameter measurement data of the user, and a plurality of load sensors (108) configured to support the seat (101) on the toilet bowl when used by the user and further to collectively generate a weight measurement data of the user, and
a fixing arrangement (106) configured to attach a lid (107) to the seat (101) and further to attach the smart toilet seat assembly to the toilet bowl, wherein the fixing arrangement (106) is configured to transfer a weight of the lid (107) exclusively to the toilet bowl and further to transfer a weight applied on the seat (101) exclusively to the plurality of load sensors (108).

2. The smart toilet seat assembly according to claim 1, wherein the plurality of physiological sensors (102, 103, 104) are arranged on a top surface (109) of the seat (101) being in physical contact with the user, and/or
wherein at least one sensor (102, 104) of the plurality of physiological sensors (102, 103, 104) is configured to be in contact with the user.

3. The smart toilet seat assembly according to claim 1 or 2,
wherein the plurality of load sensors (108) are arranged on a bottom surface (110) of the seat (101) being in contact with the toilet bowl.

4. The smart toilet seat assembly according to any of claims 1 to 3,
wherein the plurality of physiological sensors (102, 103, 104) comprise at least one electrocardiogram sensor, at least one bio-impedance sensor, at least one photoplethysmography sensor, and at least one temperature sensor, and/or
wherein the at least one physiological parameter measurement data comprises an electrocardiogram measurement data or a bio-impedance measurement data or a photoplethysmography measurement data or a temperature measurement data of the user.

5. The smart toilet seat assembly according to any of claims 1 to 4,
wherein the seat (101) comprises a switching arrangement (105) configured to switch on or switch off the plurality of physiological sensors (102, 103, 104) and/or the plurality of load sensors (108) based on a presence or an absence of the user, respectively.

6. The smart toilet seat assembly according to any of claims 1 to 5,
wherein the lid (107) comprises a receiving unit (306) configured to receive the at least one physiological parameter measurement data from the plurality of physiological sensors (102, 103, 104) and/or the weight measurement data from the plurality of load sensors (108).

7. The smart toilet seat assembly according to any of claims 1 to 6,
wherein the lid (107) comprises a processing unit (207) configured to process the at least one physiological parameter measurement data and/or the weight measurement data and further to extract one or more physiological parameters of the user from the at least one physiological parameter measurement data and/or the weight measurement data.

8. The smart toilet seat assembly according to claim 7, wherein the lid (107) comprises a display unit (305) configured to display a notification corresponding to the extracted one or more physiological parameters of the user.

9. The smart toilet seat assembly according to claim 8, wherein the lid (107) comprises a transmitting unit (303) configured to transmit the one or more physiological parameters of the user to a remote device and/or to a cloud storage (312), preferably wirelessly.

10. The smart toilet seat assembly according to any of claims 1 to 9,
wherein the seat (101) comprises a supply unit (309) configured to provide an electrical supply to the plurality of physiological sensors (102, 103, 104) and/or the plurality of load sensors (108), and
wherein the lid (107) comprises a charging unit (304) operably coupled to the supply unit (309) of the seat (101), the charging unit (304) being configured to transmit an electrical power to the supply unit (309) in order to charge or recharge the supply unit (309).

11. A load sensor (108) for generating a weight measurement data in a smart toilet seat assembly (100) according to any of claims 1 to 10, the load sensor comprises:
a holding segment (701) configured to attach the load sensor to a seat of the smart toilet seat assembly,
a foot segment (702) configured to be in contact with a toilet bowl when used by a user, and
a sensor body (703) comprising a load cell (707), the sensor body (703) being arranged between the holding segment (701) and the foot segment (702),
wherein the foot segment (702) comprises a crest (710) configured to protrude towards the holding segment (701) through the sensor body (703), and
wherein the holding segment (701) comprises a magnet (705) configured to align the crest (710) of the foot segment (702) through the sensor body (703) and further to attach the foot segment (702) with the holding segment (701).

12. The load sensor according to claim 11,
wherein the load cell (707) is a strain gauge load cell.

13. A fixing arrangement (106) for attaching a smart toilet seat assembly (100) according to any of claims 1 to 10 to a toilet bowl, the fixing arrangement comprises at least one hinge (500) comprising:
a hinge body (501, 502) configured to attach the at least one hinge to the toilet bowl,
a first bracket (503) directly coupled to the hinge body (501, 502), wherein the first bracket (503) is configured to attach a lid of the smart toilet seat assembly to the hinge body (501, 502) in order to transfer a weight of the lid exclusively to the toilet bowl and further to rotate the lid with respect to the hinge body (501, 502), and
a second bracket (506) coupled to the hinge body (501, 502) via a coupler (509), wherein the second bracket (506) is configured to attach a seat of the smart toilet seat assembly to the hinge body (501, 502) via the coupler (509) and further to rotate the seat with respect to the hinge body (501, 502),
wherein the coupler (509) is configured to move the seat along a vertical axis based on a weight applied on the seat.

14. The fixing arrangement according to claim 13,
wherein the coupler (509) comprises a limiting member (511) configured to control the movement of the seat along the vertical axis.

15. The fixing arrangement according to claim 13 or 14, wherein the first bracket (503) comprises a first damping mechanism (505) configured to control the rotational movement of the lid with respect to the hinge body (501, 502), and/or
wherein the second bracket (506) comprises a second damping mechanism (508) configured to control the rotational movement of the seat with respect to the hinge body (501, 502) .
